# EUROPEAN PATENT APPLICATION

(11) **EP 4 647 060 A1**
(43) Date of publication of application: **12.11.2025**
(21) Application number: 24175273.2
(22) Date of filing: 10.05.2024
(51) Int. Cl.: A61K 8/06, A61K 8/34, A61K 8/39, A61K 8/44, A61Q 19/00

(54) **COMPOSITION WITH EMULSIFYING PROPERTIES AND USES THEREOF**

(71) Applicant: Gobiotics BV, 4631 SL Hoogerheide (NL)
(72) Inventor: Gonry, Patrick, 2910 Essen (BE); Piotrowska, Anna, 2170 Merksem (BE)
(74) Representative: De Clercq & Partners

(57) **Abstract**

The present application provides improved compositions with emulsifying properties, and related methods and uses, wherein said compositions with emulsifying properties comprise are liquid compositions comprising (i) 8.0 - 30.0 % (w/w) of an emulsifier with a HLB value of at least 15, in particular a C16-C20 acyl glutamic acid or a salt thereof, such as sodium stearoyl glutamate; (ii) 20.0 - 60.0 % (w/w) of an alkylene glycol comprising between 3 and 7 carbon atoms, such as pentylene glycol; (iii) 20.0 - 60.0 % (w/w) water; (iv) up to 5.0 % (w/w) of an ester between polyglycerol and a C10-C14 fatty acid, such as polyglyceryl-4-laurate; and (v) up to 5.0 % (w/w) of a pH adjusting agent, such as sodium hydroxide. When the improved compositions with emulsifying properties are added to and mixed with an oily phase and an aqueous phase, stable and smooth emulsions are formed which do not require heating or the addition of stabilizing compounds.

## Description

### Field

The present application generally relates to compositions with emulsifying properties. The present application further relates to methods and uses concerning said compositions, particular to the use of said compositions in cosmetic products.

### Background

Emulsions are systems of two or more immiscible liquids or immiscible liquid phases. One of the liquid phases forms the dispersion medium or continuous phase, in which the other phase is distributed in the form of fine droplets. Most emulsions consist of water and oil or fat as immiscible phases. An o/w emulsion or oil-in-water emulsion is a fat-water mixture whose continuous phase is aqueous, whereas a w/o or water-in-oil emulsion is a oil/fat-water mixture whose continuous phase is an oil or another organic liquid that is not miscible with water. An emulsifier is generally defined as a substance that stabilizes an emulsion.

Emulsifiers are an important ingredient in many cosmetic products, acting as stabilizers to blend water and oil-based ingredients. Emulsifying compounds and compositions maintain the consistency and stability of various formulations such as lotions, creams, and serums. They generally act by reducing the surface tension between water and oil molecules, allowing them to mix evenly and prevent separation over time. They further help enhance the texture and feel of cosmetic products, providing a smooth and luxurious application experience for consumers.

Conventionally, in addition to the input of mechanical energy (mixing), the formation of an emulsion and the use of emulsifying compounds therein requires heating of the formulation. This increases the cost and carbon dioxide emissions of the production process, and may prevent or complicate the use of heat-sensitive ingredients in the emulsion.

There is thus a need for improved compositions with emulsifying properties, that allow to obtain a stable emulsion with reduced energy (heat) input.

### Summary

The inventors have developed compositions with emulsifying properties, and related methods and uses, which address one or more of the above needs. Emulsifying compositions as further described herein, particularly as further defined in the claims, said emulsifying compositions comprising an emulsifier with a HLB value of at least 15, an alkylene glycol, and water, are characterized by the formation of stable and smooth emulsions which do not require heating of the emulsion formulation, or the addition of stabilizing compounds. As the production of the emulsion does not require heating, the energy requirement and carbon dioxide emissions of the production process of the emulsified product is significantly reduced.

A first aspect of the present application provides an emulsifying composition, particularly a liquid composition with emulsifying properties, comprising
(i) 8.0 - 30.0 % (w/w) of an emulsifier with a HLB value of at least 15 or 16; particularly 10.0 - 25.0 % (w/w) of an emulsifier with a HLB value of at least 15;
(ii) 20.0 - 60.0 % (w/w) of an alkylene glycol comprising between 3 and 7 carbon atoms; particularly 25.0 - 50.0 % (w/w) of an alkylene glycol comprising between 3 and 7 carbon atoms and
(iii) 20.0 - 60.0 % (w/w) water; particularly between 25.0 - 50.0 % (w/w) water,
wherein the sum of components (i), (ii) and (ii) is at least 90 % (w/w), with % (w/w) based on the total weight of the composition.

In particular embodiments, the composition according to the present application further comprises:
(iv) up to 5.0 % (w/w), such as up to 3.0 % (w/w), of an ester between polyglycerol and a C10-C14 fatty acid; and
(v) up to 5.0 % (w/w), such as up to 3.0 % (w/w), of a pH adjusting agent, particularly wherein the pH adjusting agent is an alkali metal hydroxide;
wherein the sum of components (i)-(v) is at least 98.0 % (w/w), particularly at least 99.0 % (w/w), particularly is about 100 % (w/w).

In particular embodiments, the emulsifier with a HLB value of at least 15 or 16 is a C16-C20 acyl glutamic acid or a salt thereof, particularly stearoyl glutamic acid and/or an alkali metal salt thereof, more in particular is sodium stearoyl glutamate.

In particular embodiments, the alkylene glycol comprising between 3 and 7 carbon atoms is propylene glycol, butylene glycol or pentylene glycol, preferably is pentylene glycol.

In particular embodiments, the composition comprises (i) 10.0 - 25.0 % (w/w) of sodium stearoyl glutamate; (ii) 25.0 - 50.0 % (w/w) pentylene glycol; (iii) 25.0 - 50.0 % (w/w) water; (iv) 0.0 - 3.0 % (w/w) polyglyceryl-4-laurate, particularly 0.1 - 2.5 % (w/w) polyglyceryl-4-laurate; and (v) 0.0 - 2.0 % (w/w) sodium hydroxide, particularly 0.1 - 2.0 % (w/w) sodium hydroxide; wherein the sum of components (i), (ii) and (ii) is at least 90 % (w/w) and wherein the sum of components (i)-(v) is at least 98.0 % (w/w), particularly at least 99.0 % (w/w), particularly is about 100 % (w/w), with % (w/w) based on the total weight of the composition.

In particular embodiments, the composition has a pH value of 5.0 to 12.0, particularly a pH value of 6.0 to 12.0 or pH 8.0 to 11.0.

A further, related aspect of the present application provides a cosmetic product in the form of an oil in water emulsion, comprising 1-80% of an oil phase, an aqueous phase, and a composition according to the present application, as specified elsewhere herein, particularly wherein the cosmetic product comprises between 1.0 and 10.0 % (w/w) of a composition according to the present application, with % (w/w) based on the total weight of the cosmetic product. In particular embodiments, the cosmetic product is a cream, a lotion, a spray, or a butter.

A further related aspect of the present application provides a method for producing an emulsifying composition according to the present application, as specified elsewhere herein, said method comprising the steps of:
a) preparing a solution of 20.0 - 60.0 % (w/w), particularly 25.0 - 50.0 % (w/w) water; 20.0 - 60.0 % (w/w), particularly 25.0 - 50.0 % (w/w) of an alkylene glycol comprising between 3 and 7 carbon atoms, and; optionally, up to 5.0 % (w/w) of an ester between polyglycerol a C10-C14 fatty acid and/or up to 5.0 % (w/w) of a pH adjusting agent;
b) adding 8.0 - 30.0 % (w/w), particularly 10.0 - 25.0 % (w/w) of an emulsifier with a HLB value of at least 15 or 16 to the solution obtained in step a).

In particular embodiments, steps a) and b) are performed at a temperature of at most 40 °C, such as between 15 °C and 30 °C.

In particular embodiments, step a) comprises adding, in order, water, the ester between polyglycerol and a C10-C14 fatty acid, the alkylene glycol comprising between 3 and 7 carbon atoms, and the pH adjusting agent.

In particular embodiments, the emulsifier with a HLB value of at least 15 is a C16-C20 acyl glutamic acid or a salt thereof, particularly stearoyl glutamic acid and/or an alkali metal salt thereof, more in particular is sodium stearoyl glutamate; and/or
the alkylene glycol comprising between 3 and 7 carbon atoms is propylene glycol, butylene glycol or pentylene glycol, preferably is pentylene glycol; and/or
the ester between a polyglycerol and a C10-C14 fatty acid is polyglyceryl-4-laurate; and/or
the pH adjusting agent is an alkali metal hydroxide, such as sodium hydroxide.

A further related aspect relates to a method for preparing a cosmetic product in the form of an oil-in-water emulsion, comprising mixing and emulsifying an oil phase, an aqueous phase and an emulsifying composition according to the present application, as specified elsewhere herein, particularly wherein the mixing and emulsifying are performed at ambient temperatures, or, stated differently, a temperature of at most 40 °C , such as between 15 °C and 30 °C.

A further related aspect of the present application relates to the use of a composition according to the present application, as specified elsewhere herein, as an emulsifier, particularly as an o/w emulsifier, particularly for producing an emulsion, particularly an oil-in-water emulsion, at ambient temperatures or, stated differently, at a temperature of at most 40 °C, particularly wherein the emulsion is an emulsified cosmetic product.

### Detailed description

Before the present system and method of the invention are described, it is to be understood that this invention is not limited to particular systems and methods or combinations described, since such systems and methods and combinations may, of course, vary. It is also to be understood that the terminology used he-rein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. It is to be understood that other embodiments may be utilised and structural or logical changes may be made without departing from the scope of the present invention. The following detailed description, therefore, is not to be taken in a limiting sense, and the scope of the present invention is defined by the appended claims.

In the present application, the percentages are given by weight, unless otherwise stated.

The inventors have developed improved compositions with emulsifying properties, particularly liquid and transparent compositions with emulsifying properties. The composition with emulsifying properties according to the present application, generally comprises an emulsifier with a HLB value of at least 15, an alkylene glycol and water, and, when combined with water- and oil-based ingredients, results in the formation of stable, high-quality emulsions at room temperature, i.e. without heating or without the addition of further stabilizing compounds.

A first aspect of the present application provides an emulsifying composition, particularly a transparent, liquid composition with emulsifying properties, comprising
(i) 8.0 - 30.0 % (w/w) of an emulsifier with a HLB value of at least 15; particularly 10.0 - 25.0 % (w/w) of an emulsifier with a HLB value of at least 15, particularly an emulsifier with a HLB value of at least 16;
(ii) 20.0 - 60.0 % (w/w) of an alkylene glycol comprising between 3 and 7 carbon atoms; particularly 25.0 - 50.0 % (w/w) of an alkylene glycol comprising between 3 and 7 carbon atoms and
(iii) 20.0 - 60.0 % (w/w) water; particularly between 25.0 - 50.0 % (w/w) water,
wherein the sum of components (i), (ii) and (ii) is at least 90 % (w/w), with % (w/w) based on the total weight of the composition.

Optionally, the composition with emulsifying properties according to the present application may further comprise:
(iv) up to 5.0 % (w/w), such as up to 3.0 % (w/w), of an ester between polyglycerol and a C10-C14 fatty acid; and/or
(v) up to 5.0 % (w/w), such as up to 3.0 % (w/w), of a pH adjusting agent, particularly wherein the pH adjusting agent is an alkali metal hydroxide;
particularly wherein the sum of components (i)-(v) is at least 95.0 % (w/w), more in particular is at least 98.0 % (w/w) or at least 99.0 % (w/w), even more in particular is about 100 % (w/w).

As used herein, "HLB" refers to the "hydrophilic-lipophilic balance" associated with emulsifiers. In particular, the "HLB value" relates to the ratio of hydrophilic groups and lipophilic groups in emulsifiers, and also relates to solubility of the emulsifiers. Emulsifiers with lower HLB values are more soluble in oils and are generally more appropriate for use in water-in-oil (W/O) emulsions. Emulsifiers with higher HLB values are more soluble in water and are generally more appropriate for oil-in-water (O/W) emulsions. The HLB value of a molecule may be calculated based on the molecular structure, in particular from the weight percentage of the hydrophilic groups to the hydrophobic groups in the molecule, as known by the skilled person, or may be determined empirically as known by the skilled person, with values typically ranging from 1-20.

Emulsifiers with a HLB value of at least 15 or 16 are known in the art. In certain embodiments, said emulsifiers with a HLB value of at least 15 or 16 include fatty alcohols corresponding to formula R-OH, fatty acids corresponding to the formula R-COOH, or esters or amino acid derivatives thereof, wherein R denotes a saturated or unsaturated hydrocarbon moiety having from 12 to 22 carbon atoms, preferably from 14 to 20 carbon atoms or from 16 to 20 carbon atoms. Examples include, for instance, stearic acid/alcohol, oleic acid/alcohol, behenyl acid/alcohol, or cetyl alcohol. In particularly preferred embodiments, the emulsifier with a HLB value of at least 15 or 16 is an amino acid derivative of a C16-C20 fatty acid, in particular is an aspartic acid or glutamic acid derivative of a C16-C20 fatty acid or a salt thereof. More in particular, the emulsifier with a HLB value of at least 15 or 16 is a C16-C20 acyl glutamic acid or a salt thereof, particularly lauroyl or stearoyl glutamic acid and/or an alkali metal salt thereof. In particularly preferred embodiments, the emulsifier with a HLB value of at least 15 or 16 comprises or is sodium stearoyl glutamate.

The term "alkylene glycol" refers to, and is used synonymously with, an alkane diol, i.e. a chemical compound composed of a linear or branched alkane chain substituted with two hydroxyl groups. The composition according to the present application thus comprises a linear or branched alkane chain comprising between 3 and 7 carbon atoms substituted with two hydroxyl groups. In certain embodiments, the alkane diol is 1,2-propanediol; 1,3-propanediol; 1,2-butanediol; 1,4-butanediol; 1,2-pentanediol or 1,5-pentanediol. In particular embodiments, the alkylene glycol comprising between 3 and 7 carbon atoms is propylene glycol, butylene glycol or pentylene glycol, preferably is pentylene glycol.

In certain embodiments, the composition according to the present application comprises up to 5.0 % (w/w) or up to 3.0 % (w/w), more in particular between 0.0 and 2.5 % (w/w) or between 0.1 and 2.5 % (w/w) of an ester between polyglycerol and a C10-C14 fatty acid. In particular embodiments, the ester between polyglycerol and a C10-C14 fatty acid is an ester between polyglycerol -4 or tetraglycerol and a C10-C14 fatty acid. As used herein, the term "polyglycerol" refers to a polymer or oligomer consisting of glycerol (glycerine) monomers. polyglycerol-4 comprises on average 4 glycerol monomers. In particularly preferred embodiments, the ester between polyglycerol and a C10-C14 fatty acid is polyglyceryl-4-laurate.

In particular embodiments, the pH of the composition with emulsifying properties according to the present application ranges between 5.0 and 12.0, particularly between 6.0 and 12.0 or between 8.0 and 11.0. The pH of the compositions may be adjusted using a pH adjusting agent. In certain embodiments, the composition according to the present application comprises up to 5.0 % (w/w) or up to 3.0 % (w/w), more in particular between 0.0 and 2.0 % (w/w) or between 0.1 and 2.0 % (w/w) of a pH adjusting agent. Said pH adjusting agent may be selected from conventional acids, bases and buffer systems. In particular embodiments, the pH adjusting agent is an alkali metal hydroxide, such as sodium hydroxide or potassium hydroxide.

In particular embodiments, the emulsifying composition according to the present application is free of ethoxylated compounds, particularly free of ethoxylated emulsifiers. "Free" as used herein means that while it is preferred that the specific component is absent in the composition, i.e. 0 % (w/w), it is possible to have very small or trace amounts of it in the compositions of the invention, i.e. wherein the specific component is present as an impurity, in particular is present in an amount less than about 0.1% by weight, typically less than about 0.05% by weight, based on the total weight of the composition.

The emulsifying compositions as envisaged herein provide a natural alternative to compositions comprising ethoxylated compounds, and they are not considered as a source of microplastic. The term "natural" as used herein indicates an isolated compound or ingredient which remains essentially unchanged from its natural state, or which has undergone some limited processing to improve its stability or efficacy.

A particularly preferred composition comprises (i) 10.0 - 25.0 % (w/w) of stearoyl glutamic acid and/or sodium stearoyl glutamate; (ii) 25.0 - 50.0 % (w/w) pentylene glycol; (iii) 25.0 - 50.0 % (w/w) water; (iv) 0.0 - 3.0 % (w/w) polyglyceryl-4-laurate; and (v) 0.0 - 2.0 % (w/w) sodium hydroxide; wherein the sum of components (i)-(iii) is at least 90 % (w/w) or at least 95 % (w/w) and wherein the sum of components (i)-(v) is at least 98.0 % (w/w), particularly at least 99.0 % (w/w), more particularly is about 100 % (w/w), with % (w/w) based on the total weight of the composition.

Another particularly preferred composition comprises (i) 10.0 - 25.0 % (w/w) of sodium stearoyl glutamate; (ii) 25.0 - 50.0 % (w/w) pentylene glycol; (iii) 25.0 - 50.0 % (w/w) water; (iv) 0.1 - 3.0 % (w/w) polyglyceryl-4-laurate; and (v) 0.1 - 2.0 % (w/w) sodium hydroxide; wherein the sum of components (i)-(iii) is at least 90 % (w/w) or at least 95 % (w/w) and wherein the sum of components (i)-(v) is at least 98.0 % (w/w), particularly at least 99.0 % (w/w), more particularly is about 100 % (w/w), with % (w/w) based on the total weight of the composition.

A further, related aspect of the present application provides a method for producing the composition with emulsifying properties according to the present application, as described elsewhere herein, wherein said method generally comprises the steps of adding an emulsifier with a HLB value of at least 15, an alkylene glycol and water. More in particular, the method comprises the steps of:
a) preparing a solution of 20.0 - 60.0 % (w/w), particularly 25.0 - 50.0 % (w/w) water; 20.0 - 60.0 % (w/w), particularly 25.0 - 50.0 % (w/w) of an alkylene glycol comprising between 3 and 7 carbon atoms, and; optionally, up to 5.0 % (w/w) of an ester between polyglycerol and a C10-C14 fatty acid and/or up to 5.0 % (w/w) of a pH adjusting agent;
b) adding 8.0 - 30.0 % (w/w), particularly 10.0 - 25.0 % (w/w) of an emulsifier with a HLB value of at least 15 to the solution obtained in step a);
wherein the sum of the amounts of water, alkylene glycol and the emulsifier with a HLB value of at least 15 is at least 90 % (w/w) or at least 95 % (w/w) and wherein the sum of the amounts of water, alkylene glycol, pH adjusting agent, the ester between polyglycerol and a C10-C14 fatty acid, and the emulsifier with a HLB value of at least 15 is at least 98.0 % (w/w), particularly at least 99.0 % (w/w), more particularly is about 100 % (w/w), with % (w/w) based on the total weight of the composition..

In particularly preferred embodiments, the method comprises the steps of:
a) preparing a solution of 25.0 - 50.0 % (w/w) water; 25.0 - 50.0 % (w/w) of propylene glycol, butylene glycol or pentylene glycol, particularly 25.0 - 50.0 % (w/w) of pentylene glycol; between 0.0 and 3.0 % (w/w) or between 0.0 and 2.5 % (w/w) of polyglyceryl-4-laurate; and between 0.0 and 3.0 % (w/w) or between 0.0 and 2.0 % (w/w) of sodium hydroxide;
b) adding 10.0 - 25.0 % (w/w) of stearic glutamic acid and/or an alkali metal salt thereof, particularly sodium stearic glutamate, to the solution obtained in step a).

In particularly preferred embodiments, step a) of the method comprises adding, in order, water, the ester between polyglycerol and a C10-C14 fatty acid, the alkylene glycol comprising between 3 and 7 carbon atoms, and the pH adjusting agent.

Advantageously, the composition with emulsifying properties according to the present application can be prepared without heating, in particular at room temperature. Room temperature or ambient temperature is understood as meaning temperatures of at most 40 °C, such as between such as between 10 °C and 35 °C or between 15 °C and 30 °C. Accordingly, in particular embodiments, steps a) and b) of the method are performed at a temperature of at most 40 °C, such as between 10 °C and 35 °C or between 15 °C and 30 °C.

The composition with emulsifying properties according to the present application is particularly suitable for the cold preparation, i.e. at room temperature, of oil-in-water emulsions, particularly for the cold preparation of cosmetic products in the form of oil-in-water emulsions.

A further related aspect of the present application thus provides for a product or formulation in the form of an oil-in-water emulsion, particularly a cosmetic product of formulation in the form of an oil-in-water emulsion, comprising 1-80% of an oily phase, such as 10-80% of an oily phase, an aqueous phase, and a composition with emulsifying properties according to the present application, as described elsewhere herein, particularly comprising between 1.0 and 10.0 % (w/w) of a composition with emulsifying properties according to the present application, with % (w/w) based on the total weight of the product.

In particular embodiments, the product, particularly cosmetic product, in the form of an oil-in-water emulsion according to the present application, is a skin care cosmetic product, in particular a cream, a lotion, such as a cleansing lotion, a butter, or a spray.

It is understood that, in such products, particularly cosmetic products, in the form of an oil-in-water emulsion, the composition with emulsifying properties according to the present application is used together and combined with additional ingredients or adjuvants known to the skilled person, particularly with cosmetically acceptable additional ingredients or adjuvants known to the skilled person. It is further understood that these ingredients may be introduced into or are present in the oily phase or the aqueous phase of the product according to the present application, depending on their hydrophilic or lipophilic nature. The water phase comprises all substances of the product or formulation which are added to or dissolved in it due to their hydrophilic properties. Likewise, the oil phase comprises the oils, fats and wax components of the oil-in-water emulsion, and the other substances of the product or formulation which are added to or dissolved in the oil phase do to their lipophilic or hydrophobic properties.

The additional ingredients or adjuvants may comprise one or more of oils, fats or waxes; emulsifiers, particularly emulsifiers different from the compounds in the composition according to the present application; surfactants; thickeners, such as polymer-based thickeners, particularly polysaccharides, such as starch and starch derivatives and natural gums, such as xanthan gum, guar gum, carrageenan and cellulose gum; preservatives; fillers; solvents, such as water, ethanol or glycerine and the like; perfumes, pigments or dyes; and/or other active or functional ingredients, such as UV protective filters, antioxidants, anti-acne agents, anti-wrinkle agents, amino acids, peptides, proteins, vitamins, essential oils, and the like. These components are present in the product, particularly cosmetic product, in the form of an oil-in-water emulsion, in amounts as conventionally used.

The oils, fats or waxes present in the oily phase of the oil-in-water emulsion are not particularly limited, and may be a mineral oil, such as paraffin oil or vaseline oil; an oil of vegetable origin, such as sweet-almond oil, jojoba oil, coconut oil, olive oil, argan oil, shea butter, grapeseed oil, avocado oil, sunflower oil, macadamia oil, rapeseed oil, or castor oil; a modified vegetable oil, an oil of animal origin, or a synthetic oil. Natural fatty materials, i.e. non-synthetic oils, fats or waxes, are particularly preferred.

A further, related aspect of the present application provides a method for producing the product, in particular cosmetic product, in the form of an oil-in-water emulsion according to the present application, wherein said method generally comprises the step of mixing and emulsifying an oily phase, an aqueous phase and a composition with emulsifying properties according to the present application and as described elsewhere herein.

The method typically comprises the steps of (a) providing the components of the product to be emulsified, i.e. the components making up the oily phase and the aqueous phase, as described elsewhere herein; (b) providing the composition with emulsifying properties according to the present application; and producing an emulsion, particularly an oil-in-water emulsion by mixing the components of step (a) and the composition of step (b). In particular, the composition with emulsifying properties according to the present application is added in a proportion of between 1.0 and 10.0 % (w/w), based on the total weight of the emulsion. Advantageously, the product in the form of an oil-in-water emulsion can be prepared without heating, in particular at room temperature, thus reducing the energy requirements and carbon dioxide emissions of the production process. Room temperature or ambient temperature is understood as meaning temperatures of at most 40 °C, such as between such as between 10 °C and 35 °C or between 15 °C and 30 °C. Accordingly, in particular embodiments, the emulsion is produced at a temperature of at most 40 °C, such as between 10 °C and 35 °C or between 15 °C and 30 °C.

A further, related aspect of the present application relates to the use of the composition with emulsifying properties according to the present application and as described elsewhere herein, as an emulsifier, particularly as an oil-in-water emulsifier. Preferably, the the composition with emulsifying properties according to the present application is used for producing an emulsion at ambient temperatures, or stated differently, at a temperature of at most 40 °C. More in particular, said emulsion is an emulsified cosmetic product. More in particular, said emulsion is an emulsified cosmetic product for skin care applications.

### Examples

### Example 1 - Preparation of an emulsifying composition according to the present composition.

An emulsifying composition according to the present application is prepared by adding and mixing until dissolved, at room temperature, in order, the following ingredients,
(i) water (35.9 % w/w),
(ii) polyglyceryl-4 laurate (2.5 % w/w),
(iii) pentylene glycol (49 % w/w),
(iv) sodium hydroxide (1.6 % w/w), for adjustment of the pH, and
(v) sodium stearoyl glutamate (11 % w/w).

Advantageously, the preparation of this composition does not require heating the composition. It is also found that polyglyceryl-4 laurate and sodium hydroxide are optional compounds to obtain an emulsifying composition according to the present application at room temperature.

### Example 2 - Preparation of lotion comprising an emulsifying composition according to the present application.

A sample emulsifying composition according to the present application was made and is referred to as sample "Cold". It was found that this sample could emulsify up to 80% oil. The sample "cold" was compatible with vegetable oils.

A cold process, fully natural lotion, free from microplastics and silicones was prepared as follows. The ingredients are listed in Table 1.

**Table 1. Lotion ingredients**

| **Phase** | **Ingredient** | **% (w/w)** |
|---|---|---|
| A | water | QS |
| A | Sclerotium gum (Amigel SM2 granulated, Alban Muller) | 0.3 |
| A | Xantham gum (Keltrol T, from CP Kelco) | 0.05 |
| A | A mixture of cellulose gum, xanthan gum, inulin, cellulose, glucose and fructose (Prebiulin C90, from Gobiotics) | 0.45 |
| A | Sample "Cold" | 1 |
| A | Pentylene glycol (preservative) | QS |
| A | Sodium Anisate (preservative) | QS |
| B | Jojoba oil | 5 |
| B | Decyl oleate | 5 |
| B | Babassu oil (refined) | 3 |
| B | Rice bran oil (refined) | 7 |
| B | Tocopherol, in sunflower oil (Cosphaderm T-70 (antioxidant, from Cosphatec) | 0.2 |
| B | Vegetable wax (Acticire MB, from Gattefosse) | 1 |
| B | A mixture of isoamyl laurate and isoamyl cocoate | 2 |
| C | inulin | 1 |
| C | Mannose, glucose, beta-glucan, sodium hyaluronate (Tosolin P, Gobiotics) | 0.06 |
| D | NaOH / citric acid (pH adjusters) | QS |

In phase A, Amigel, xantham gum and prebiulin were mixed at room temperature, until clear. Next, the rest of ingredients of phase A are mixed (at room temperature).

The ingredients of phase B were premixed until clear (at room temperature). This B mixture was added to the A mixture and homogenized for 5 min/10 000 rpm.

Next, ingredients of phase C were added (at room temperature) and well mixed.

Finally, the pH is monitored and adjusted, if necessary, to pH 5 - 5.5.

By using the "Cold" composition as the emulsifier, the lotion could be prepared at room temperature. In contrast, in a comparative example with a similar composition, but with pure sodium stearoyl glutamate instead of the "Cold" composition as emulsifier, preparation and emulsification of the lotion require heating to about 75°C.

### Example 3 - Preparation of a body cream comprising an emulsifying composition according to the present application.

A sample emulsifying composition according to the present application was made and is referred to as sample "Cold". It was found that this sample could emulsify up to 80% oil. The sample "cold" was compatible with vegetable oils.

A combined body lotion and shower gel was prepared as follows. The ingredients are listed in Table 2.

**Table 2. body cream ingredients**

| **Phase** | **Ingredient** | **% (w/w)** |
|---|---|---|
| A | water | QS |
| A | Xantham gum (Keltrol T, from CP Kelco) | 0.4 |
| A | Glycerin | 5 |
| A | Pentylene glycol (preservative) | QS |
| A | Sample "Cold" | 10 |
| B | sunflower oil (high oleic) | 10 |
| B | Isoamyl laurate | 5 |
| C | Rice starch | 15 |
| C | Inulin, fructose | 2 |
| D | lactic acid (pH adjusters) | QS |

In phase A, the ingredients of phase A were mixed well, until clear, at room temperature. The ingredients of phase B were premixed, added to mixture A, and homogenized for 5 min/10 000 rpm, all performed at room temperature.

Next, ingredients of phase C were added (at room temperature) and well mixed.

Finally, the pH is monitored and adjusted, if necessary, to pH 4.8-5.6.

By using the "Cold" composition as the emulsifier, the body cream could be prepared at room temperature. In contrast, in a comparative example with a similar composition, but with pure sodium stearoyl glutamate instead of the "Cold" composition as emulsifier, preparation and emulsification of the body cream require heating to about 75°C.

## Claims

1. An emulsifying composition comprising
(i) 8.0 - 30.0 % (w/w) of an emulsifier with a HLB value of at least 15; particularly 10.0 - 25.0 % (w/w) of an emulsifier with a HLB value of at least 15;
(ii) 20.0 - 60.0 % (w/w) of an alkylene glycol comprising between 3 and 7 carbon atoms; particularly 25.0 - 50.0 % (w/w) of an alkylene glycol comprising between 3 and 7 carbon atoms and
(iii) 20.0 - 60.0 % (w/w) water; particularly between 25.0 - 50.0 % (w/w) water,
wherein the sum of components (i), (ii) and (ii) is at least 90 % (w/w), with % (w/w) based on the total weight of the composition.

2. The composition according to claim 1 further comprising:
(iv) up to 5.0 % (w/w), such as up to 3.0 % (w/w), of an ester between polyglycerol and a C10-C14 fatty acid; and/or
(v) up to 5.0 % (w/w), such as up to 3.0 % (w/w), of a pH adjusting agent, particularly wherein the pH adjusting agent is an alkali metal hydroxide;
wherein the sum of components (i)-(v) is at least 98.0 % (w/w), particularly at least 99.0 % (w/w), particularly is about 100 % (w/w).

3. The composition according to claim 1 or 2, wherein the emulsifier with a HLB value of at least 15 is a C16-C20 acyl glutamic acid or a salt thereof, particularly stearoyl glutamic acid and/or an alkali metal salt thereof, more in particular is sodium stearoyl glutamate.

4. The composition according to any one of claims 1 to 3 wherein the alkylene glycol comprising between 3 and 7 carbon atoms is propylene glycol, butylene glycol or pentylene glycol, preferably is pentylene glycol.

5. The composition according to any one of claims 1 to 4, wherein the composition comprises:
(i) 10.0 - 25.0 % (w/w) of sodium stearoyl glutamate.
(ii) 25.0 - 50.0 % (w/w) pentylene glycol;
(iii) 25.0 - 50.0 % (w/w) water;
(iv) 0.0 - 3.0 % (w/w) polyglyceryl-4-laurate, particularly 0.1 - 2.5 % (w/w) polyglyceryl-4-laurate; and
(v) 0.0 - 2.0 % (w/w) sodium hydroxide, particularly 0.1 - 2.0 % (w/w) sodium hydroxide,
wherein the sum of components (i), (ii) and (ii) is at least 90 % (w/w),
wherein the sum of components (i)-(v) is at least 98.0 % (w/w), particularly at least 99.0 % (w/w), particularly is about 100 % (w/w),
with % (w/w) based on the total weight of the composition.

6. The composition according to any one of claims 1 to 5, wherein the composition has a pH value of 5 to 12, particularly a pH value of 6 to 12 or 8 to 1.

7. A cosmetic product in the form of an oil in water emulsion, comprising 1-80% of an oily phase, an aqueous phase, and a composition according to any one of claims 1 to 6, particularly comprising between 1.0 and 10.0 % (w/w) of a composition according to any one of claims 1 to 6, with % (w/w) based on the total weight of the cosmetic product.

8. The cosmetic product according to claim 7, wherein the cosmetic product is a cream, a lotion, a spray, or a butter.

9. Method for producing an emulsifying composition according to any one of claims 1 to 6 comprising the steps of:
a) preparing a solution of 20.0 - 60.0 % (w/w), particularly 25.0 - 50.0 % (w/w) water; 20.0 - 60.0 % (w/w), particularly 25.0 - 50.0 % (w/w) of an alkylene glycol comprising between 3 and 7 carbon atoms, and; optionally, up to 5.0 % (w/w) of an ester between polyglycerol and a C10-C14 fatty acid and/or up to 5.0 % (w/w) of a pH adjusting agent;
b) adding 8.0 - 30.0 % (w/w), particularly 10.0 - 25.0 % (w/w) of an emulsifier with a HLB value of at least 15 to the solution obtained in step a).
wherein the sum of the water, the alkylene glycol, the ester between the polyglycerol and a C10-C14 fatty acid, the emulsifier with a HLB value of at least 15 and the pH adjusting agent is at least 98.0 % (w/w), particularly at least 99.0 % (w/w), particularly is about 100 % (w/w); and wherein the sum of the water, the alkylene glycol, and the emulsifier with a HLB value of at least 15 is at least 90 % (w/w), with % (w/w) based on the total weight of the composition.

10. The method according to claim 9, wherein steps a) and b) are performed at a temperature of at most 40 °C, such as between 15 °C and 30 °C.

11. The method according to claim 9 or 10, wherein step a) comprises adding, in order, water, the ester between polyglycerol and a C10-C14 fatty acid, the alkylene glycol comprising between 3 and 7 carbon atoms, and the pH adjusting agent.

12. The method according to any one of claims 9 to 11, wherein:
- the emulsifier with a HLB value of at least 15 is a C16-C20 acyl glutamic acid or a salt thereof, particularly stearoyl glutamic acid and/or an alkali metal salt thereof, more in particular is sodium stearoyl glutamate; and/or
- the alkylene glycol comprising between 3 and 7 carbon atoms is propylene glycol, butylene glycol or pentylene glycol, preferably is pentylene glycol; and/or
- the ester between a polyglycerol and a C10-C14 fatty acid is polyglyceryl-4-laurate; and/or
- the pH adjusting agent is an alkali metal hydroxide, such as sodium hydroxide

13. A method for preparing a cosmetic product according to claim 7 or 8 comprising mixing and emulsifying an oil phase, an aqueous phase and a composition according to any one of claims 1 to 6.

14. The method according to claim 13, wherein the method is performed at a temperature of at most 40 °C, such as between 15 °C and 30 °C.

15. Use of a composition according to any one of claims 1 to 6 as an emulsifier, particularly as an o/w emulsifier, particularly for producing an emulsion at a temperature of at most 40 °C.
